# EUROPEAN PATENT APPLICATION

(11) **EP 4 623 701 A1**
(43) Date of publication of application: **01.10.2025**
(21) Application number: 23907124.4
(22) Date of filing: 21.12.2023
(51) Int. Cl.: A23L 5/00, A23L 13/74, A23L 33/18, C12N 9/48, C12N 9/50, C12N 9/52

(54) **ENZYME COMPOSITION FOR PRODUCING FOOD**

(30) Priority: 22.12.2022 JP 2022205562
(71) Applicant: Nagase Viita Co., Ltd., Okayama-shi, Okayama 702-8006 (JP)
(72) Inventor: MIZOBUCHI, Hisanori, Okayama-shi, Okayama 702-8006 (JP); NAKAHIGASHI, Ryota, Okayama-shi, Okayama 702-8006 (JP)
(74) Representative: Manitz Finsterwald Patent- und Rechtsanwaltspartnerschaft mbB
(86) International application number: PCT/JP2023/045841
(87) International publication number: WO 2024/135764

(57) **Abstract**

The present invention provides a collagenase enzyme composition with high storage stability that can be used in food production processes while reducing the use of stabilizers and antiseptic agents. The present invention relates to an enzyme composition for producing a food, containing a collagenase and having substantially no contaminating activity.

## Description

### TECHNICAL FIELD

The present invention relates to an enzyme composition for producing a food.

### BACKGROUND ART

Collagen is a protein found in the skin, tendons, bones, and cartilage of animals, and it accounts for the largest proportion of all proteins in their bodies. Collagen peptides, which are collagen degradation products, are products obtained by degrading collagen to structural units consisting of three amino acids, including glycine. Collagen peptides are highly absorbable into the body and are therefore widely used in food, beverages, supplements, etc. Moreover, collagen peptides are also used as cosmetic components due to their moisturizing properties.

Patent Literatures 1 and 2 each disclose a method for producing collagen peptides using a collagenase that degrades collagen in a sequence-specific manner. Patent Literatures 3 and 4 each disclose the use of a collagenase to produce casings for processed meat products or to tenderize meat.

### CITATION LIST

### - Patent Literature

Patent Literature 1: WO 2021/200955
Patent Literature 2: JP 2017-537978 A
Patent Literature 3: JP 2020-074697 A
Patent Literature **4:** JP 2016-052304 A

### SUMMARY OF INVENTION

### - Technical Problem

Conventional collagenase enzyme compositions used in food production processes have insufficient storage stability. Generally, stabilizers or antiseptic agents may be added to enzyme preparations to maintain the enzymatic activity or to provide antisepsis. However, the addition of stabilizers or antiseptic agents will reduce the enzymatic activity per weight unit of the enzyme composition. Moreover, since such chemical substances fall under the category of designated additives with restricted use in food applications, they tend to be avoided in the market due to growing concerns about food safety. The present invention aims to provide a collagenase enzyme composition with high storage stability that can be used in food production processes while reducing the use of stabilizers and antiseptic agents.

### - Solution to Problem

The present invention relates to an enzyme composition for producing a food, containing a collagenase and having substantially no contaminating activity.

The contaminating activity is preferably protease activity.

The enzyme composition preferably has a P/C ratio of 2.0 or lower, where P represents protease activity (U/g) and C represents collagenase activity (U/g).

The collagenase is preferably derived from a bacterium.

A residual collagenase activity of the enzyme composition is preferably 50% or higher after storage at 40°C for one month.

The food is preferably a collagen peptide or a processed meat food.

The present invention also relates to a food production method, including processing a food material using the enzyme composition.

The present invention also relates to a food, containing the enzyme composition.

### - Advantageous Effect of Invention

The collagenase enzyme composition of the present invention has high storage stability and can reduce the cost for food production.

### BRIEF DESCRIPTION OF DRAWINGS

FIG. 1 shows collagenase activities after storage at 5°C.
FIG. 2 shows collagenase activities after storage at 15°C.
FIG. 3 shows collagenase activities after storage at 25°C.
FIG. 4 shows the results of a meat tenderizing test.

### DESCRIPTION OF EMBODIMENTS

### <Enzyme composition>

The present invention relates to an enzyme composition for producing a food, containing a collagenase and having substantially no contaminating activity.

### <Collagenase>

The collagenase is an enzyme that degrades collagen to structural units composed of tripeptides containing glycine. Examples of the tripeptides containing glycine include glycine-proline-hydroxyproline, glycine-proline-alanine, and glycine-alanine-hydroxyproline.

The collagenase can be derived from any origin, for example, a microorganism, an animal, or a plant. In view of easy availability, the collagenase is preferably derived from a microorganism. The microorganism may be a bacterium or a fungus, preferably a bacterium.

The bacterium may be an actinomycete or *Bacillus subtilis.* Examples of the actinomycete include those of the genera *Streptomyces, Actinomyces, Nocardia,* and *Rhodococcus.* Examples of the *Bacillus subtilis* include those of the genera *Bacillus* and *Lysinibacillus.* The fungus may be a filamentous fungus, a basidiomycete, or a yeast. Of these, the collagenase is preferably derived from a bacterium, more preferably an actinomycete, still more preferably the genus *Streptomyces,* particularly preferably *Streptomyces violaceoruber.* A collagenase preparation, which is a modifier according to the present invention, may contain only one type or two or more types of such collagenases.

The collagenase may be obtained by purification from a plant, an animal, or a microorganism from which it is derived, or by large-scale production using gene recombination techniques followed by purification. Moreover, the collagenase may be a wild-type collagenase or a mutant collagenase.

When the collagenase accumulates in the cells of the source organism, the collagenase may be obtained by crushing the tissues and cells followed by centrifugation or the like to obtain a cell-free extract. The cell-free extract, as a starting material, may be purified, if necessary, by appropriately combining general protein purification methods such as salting-out, ion exchange chromatography, gel filtration chromatography, hydrophobic chromatography, and affinity chromatography. When the collagenase is produced by extracellular secretion from a microorganism, it can be purified from the medium.

The collagenase activity in the enzyme composition of the present invention is preferably 10 units (U) or more, more preferably 50 units (U) or more, still more preferably 100 units (U) or more per gram of the enzyme composition. A higher upper limit is preferred, without any limitation. Generally, the upper limit is 5000 units (U) or less per gram of the enzyme composition. Moreover, the weight of the collagenase in the enzyme composition of the present invention is preferably 0.01 to 20% by weight, more preferably 0.1 to 10% by weight per gram of the enzyme composition. Here, the collagenase activity can be determined according to the measurement method described in ANALYTICAL BIOCHEMISTRY, 136, pp. 446-450 (1984), in which an enzyme is added to an AZO collagen aqueous solution (0.9% AZOCOLL Substrate available from Sigma-Aldrich) and reacted at 37°C and pH 7.5 for 10 minutes, and the increase in OD at 595 nm per minute is defined as one unit (U).

### <Contaminating activity>

The enzyme composition has substantially no contaminating activity. The contaminating activity is an enzymatic activity other than collagenase activity that could impair collagenase activity, such as protease activity, amylase activity, or lipase activity. Of these, the enzyme composition preferably has substantially no protease activity. Here, the phrase "has substantially no contaminating activity" means that the residual collagenase activity of the enzyme composition is 50% or higher after storage at 40°C for one month. The residual collagenase activity is preferably 60% or higher, more preferably 65% or higher.

It should be noted that the term "protease activity" may be interpreted as encompassing all protein hydrolysis activity. In the present invention, however, contaminating protease activity refers to protein hydrolysis activity other than collagenase activity.

Any method can be used to allow the enzyme composition to have substantially no contaminating activity. Examples include a method using a collagenase that originally has low contaminating activity, a method using a collagenase whose contaminating activity has been reduced by mutation, a method that reduces contaminating activity through purification, and a method that reduces contaminating activity by incorporating an optional component.

With regard to contaminating protease activity in the enzyme composition, the P/C ratio, where P represents protease activity (U/g) and C represents collagenase activity (U/g), is preferably 3.5 or lower, more preferably 3 or lower, still more preferably 2.5 or lower, further preferably 2 or lower, particularly preferably 1.5 or lower. Here, the protease activity can be determined according to the measurement method described in the ninth edition of the Japan's Specifications and Standards for Food Additives, 2018, pp. 887-888, in which an enzyme is added to a milk casein aqueous solution (0.6% casein, bovine milk, free of carbohydrate and fatty acid, Merck) and reacted at 30°C and pH 7.5 for 10 minutes, and the amount of the enzyme that produces an increase in Folin reagent-reactive substances equivalent to 1 µg of L-tyrosine per minute is defined as 1 U.

The enzyme composition of the present invention has substantially no contaminating activity and therefore has high storage stability. The residual collagenase activity of the enzyme composition is preferably 50% or higher, more preferably 60% or higher, still more preferably 65% or higher, after storage at 40°C for one month.

### <Optional component>

The enzyme composition may contain, in addition to the collagenase, other components that can usually be contained in enzyme compositions, to the extent that the effect of the present invention is not impaired. Examples of such components include excipients, pH adjusters, preservatives, thickening polysaccharides, emulsifiers, inorganic salts, amino acids, peptides or proteins, and solvents. The amounts of these components are not limited and may be selected in any amount by those skilled in the art. Note that the enzyme composition does not have to contain a pH adjuster, a preservative, a thickening polysaccharide, an emulsifier, an inorganic salt, an amino acid, or a peptide or protein to solve the problem in the present application, as illustrated in EXAMPLES.

Examples of excipients include dextrin, trehalose, and grain flour such as rice flour or wheat flour.

Examples of pH adjusters include ascorbic acid, acetic acid, dehydroacetic acid, lactic acid, citric acid, gluconic acid, succinic acid, tartaric acid, fumaric acid, malic acid, and adipic acid as well as sodium (Na) salts, calcium (Ca) salts, or potassium (K) salts of these organic acids; and carbonic acid, phosphoric acid, and pyrophosphoric acid as well as sodium (Na) salts or potassium (K) salts of these inorganic acids.

Examples of preservatives include propionic acid, salts of propionic acid, salts of sulfurous acid, salts of benzoic acid, sorbic acid, and salts of sorbic acid. Examples of the salts include sodium (Na) salts, calcium (Ca) salts, potassium (K) salts, and polyamines.

Examples of thickening polysaccharides include modified starches, gums, alginic acid, alginic acid derivatives, pectin, carrageenan, curdlan, pullulan, gelatin, cellulose derivatives, agar, tamarind, psyllium, and glucomannan.

Examples of emulsifiers include glycerin fatty acid esters, polyglycerin fatty acid esters, sucrose fatty acid esters, propylene glycol fatty acid esters, sorbitan fatty acid esters, lecithin, enzymatically degraded lecithin, and saponin.

Examples of inorganic salts include sodium chloride, ammonium sulfate, sodium sulfate, calcium chloride, and polymeric phosphates.

Examples of amino acids include aspartic acid, threonine, serine, asparagine, glutamic acid, glutamine, proline, glycine, alanine, valine, cystine, methionine, isoleucine, leucine, tyrosine, phenylalanine, histidine, lysine, tryptophan, and arginine.

Examples of peptides or proteins include collagen peptides, milk proteins, soybean proteins, casein proteins, egg proteins, wheat proteins, maize proteins, buckwheat proteins, proteins derived from seaweeds or microalgae, pea proteins, animal meat proteins, seafood proteins, yeasts and yeast-derived proteins, enzymatic decomposition products of these proteins, fish meat peptides, soy peptides, whey peptides, and sardine peptides. These peptides or proteins can contribute to the storage stability of the collagenase by serving as substrates for contaminating activity or by functioning as inhibitors of contaminating activity.

Examples of solvents include water and polyhydric alcohols. Specific examples of the polyhydric alcohols include glycerol, sorbitol, propylene glycol, poly(vinyl alcohol), pentaerythritol, ethylene glycol, diethylene glycol, triethylene glycol, and polyethylene glycol. Each of these solvents may be used alone, or two or more of these may be used in admixture. Preferred solvent mixtures are mixtures of water and solvents other than water, with mixtures of water and glycerol being more preferred. The weight ratio of [water]:[solvent other than water] is preferably 2:1 to 1:8, more preferably 1:1 to 1:4. Moreover, when a solvent is used, the weight ratio of enzyme:solvent is preferably 2:1 to 1:2.

The enzyme composition may be in any form, such as liquid, paste, powder, or granules. The enzyme composition in liquid or paste form can be greatly affected by contaminating activity and therefore tends to show significantly improved storage stability by having substantially no contaminating activity. The enzyme composition in powder or granule form tends to be less affected by contaminating activity.

The enzyme composition in liquid or paste form preferably has a pH of 5.5 to 9.0, more preferably 5.5 to 6.5.

### <Method for producing enzyme composition>

The enzyme composition may be produced by any method that can produce an enzyme composition containing a collagenase and having substantially no contaminating activity. For example, a solid enzyme composition can be produced by dissolving a collagenase in a solvent such as water and then freeze-drying the solution to obtain an enzyme powder. If necessary, the enzyme powder may be mixed with an optional component using a mixer. An optional component may also be dissolved simultaneously when the collagenase is dissolved in a solvent such as water. Examples of the mixer include container tumbling mixers, stationary mixers, and combined mixers. The mixer may be appropriately selected depending on the target activity level and amount and the type of excipient. A liquid enzyme composition can be produced by dissolving an enzyme and an optional component in a solvent.

### <Food production>

The enzyme composition of the present invention can be used in food production. The food may be any food that can be produced from a collagen-containing food material. Examples of the food include collagen peptides, processed meat foods, and processed fish meat foods. Examples of the processed meat foods include sausages, hamburger patties, aged meat, and meat extract. Examples of the processed fish meat foods include fish sausage called "Chikuwa", fish cake called "Kamaboko", simmered fish, and fish extract.

Specific examples of collagen peptides that can be produced using the enzyme composition of the present invention include glycine-proline-hydroxyproline, glycine-proline-alanine, and glycine-alanine-hydroxyproline. The collagen peptides produced using the enzyme composition of the present invention can be suitably used as active ingredients of functional foods, functional beverages, cosmetics, or pharmaceuticals.

Processed meat foods produced using the enzyme composition of the present invention provide improved texture due to the degradation of collagen or gelatin. For example, when a food high in collagen or gelatin is cooled, jellied broth may form, deteriorating the texture. In contrast, treating such a food with the enzyme composition of the present invention can prevent the formation of jellied broth.

The food production can be performed by a method including processing a food material using the enzyme composition. In the step of processing a food material using the enzyme composition, the collagen contained in the food material is treated with the collagenase in the enzyme composition.

The food material may be any food material that contains collagen. Examples include bones, skin, tendons, and cartilage from animals, and bones, skin, meat, cartilage, and scales from fish. Examples of animals include cattle, pigs, and chickens. Examples of fish include tuna, sea bream, mackerel, horse mackerel, sardine, salmon, bonito, yellowtail, saury, and tilapia. Collagen, or its partially degraded form gelatin, purified from these materials may be used to produce collagen peptides using the enzyme composition of the present invention.

The treatment with the collagenase may be carried out under any condition. The temperature is preferably 30°C to 60°C, more preferably 40°C to 50°C. The pH is preferably 6.0 to 9.0, more preferably 7.0 to 8.0. The treatment duration is preferably 1 to 24 hours.

A specific example of a method for processing a food material using the enzyme composition of the present invention includes treating the food material, preferably meat, with a pickling liquid containing the enzyme composition of the present invention. The collagenase activity in the pickling liquid is preferably 0.001 to 100 U/mL, more preferably 0.005 to 50 U/mL, still more preferably 0.01 to 10 U/mL. The pickling liquid may further contain a salt such as sodium chloride, sodium hydrogen carbonate, sodium nitrate, sodium nitrite, magnesium chloride, calcium chloride, calcium lactate, potassium nitrate, or potassium nitrite; or a thickening polysaccharide such as a modified starch, gum, alginic acid, alginic acid derivative, pectin, carrageenan, curdlan, pullulan, gelatin, cellulose derivative, agar, tamarind, psyllium, or glucomannan, preferably at a concentration of 0.01 to 100% by weight, more preferably 0.05 to 50% by weight. The amount of the collagenase used in the enzyme composition for meat is preferably such that the collagenase activity per gram of meat is 0.0005 to 50 U, more preferably 0.002 to 5 U.

The treatment of meat with the pickling liquid may be carried out by any method that can bring the pickling liquid into contact with the meat. Examples include spraying the pickling liquid onto the meat, immersing the meat in the pickling liquid, and injecting the pickling liquid into the meat using an injector. The duration of treatment with the pickling liquid is preferably within 100 hours, more preferably 1 to 80 hours. The temperature in the treatment with the pickling liquid is preferably 2°C to 20°C, more preferably 5°C to 15°C. Before or after the treatment with the pickling liquid, the meat may be physically tenderized by using a tenderizer, a tumbler, or similar equipment, or by making slits with a knife or similar tool.

In the production of collagen peptides using the enzyme composition of the present invention, treatment with the collagenase may optionally be followed by purification of the collagen peptides through filtration, ion exchange, activated carbon treatment, or other techniques. Moreover, purification of the collagen peptides is not necessarily required. Ingesting a mixture of the collagen peptides and the enzyme composition does not cause any problems. Like the collagen peptides, the ingested enzyme composition will be digested and absorbed in the body. If the collagenase needs to be deactivated, the food material treated with the collagenase may be heated at 70°C or higher.

### EXAMPLES

The present invention is described below with reference to examples, but the present invention is not limited thereto. Hereinafter, the units "part(s)" and "%" refer to "part(s) by weight" and "% by weight", respectively, unless otherwise specified.

The following enzymes were used in the tests described below. The compounding amounts (%) described below are expressed by weight for each substance.
- Collagenase available from Nagase ChemteX Corp., NAGAZYME-01, 210 units (U)/g
- Protease available from Nagase ChemteX Corp., product name: BIOPRASE SP-20FG, 100,000 units (U)/g

### (1) Example 1, Comparative Examples 1 to 3

### (1-1) Production of enzyme composition

The collagenase was added to a 50% glycerol solution such that it had the activity shown in Table 1 to produce a liquid enzyme composition. In Comparative Examples 1 to 3, the protease was also added in addition to the collagenase.

### (1-2) Accelerated stability test

The enzyme compositions were stored at 40°C for one month. The activity of the enzyme compositions was measured before and after storage as described below, and the relative activity was calculated based on the activity immediately after production, taken as 100%. Table 1 shows the results.

**[Table 1]**

| | Example 1 | Comparative Example 1 | Comparative Example 2 | Comparative Example 3 |
|---|---|---|---|---|
| Collagenase activity C (U/g) | 219 | 213 | 224 | 276 |
| Protease activity P (U/g) | 291 | 844 | 5058 | 41059 |
| P/C ratio | 1.3 | 4.0 | 22.6 | 148.7 |
| Residual activity (%) after accelerated stability test | 66.9% | 16.4% | 2.3% | 28.2% |

The collagenase activity was determined by reacting the collagenase with an AZO collagen (azo dye-impregnated collagen) as a substrate at 37°C and pH 7.5 for 10 minutes, with the increase in OD at 595 nm per minute defined as 1 U.

As shown in Table 1, the residual activity decreased to approximately 30% or lower after the accelerated test in Comparative Examples 1 to 3. In Example 1, the residual activity was maintained at 65% or higher.

### (2) Example 2, Comparative Examples 4 to 6

### (2-1) Production of enzyme composition

The collagenase was added to a 50% glycerol solution such that it had the activity shown in Table 2 to produce a liquid enzyme composition. In Comparative Examples 4 to 6, the protease was also added in addition to the collagenase.

**[Table 2]**

| | Example 2 | Comparative Example 4 | Comparative Example 5 | Comparative Example 6 |
|---|---|---|---|---|
| Collagenase activity C (U/g) | 186 | 186 | 185 | 177 |
| Protease activity P (U/g) | 253 | 712 | 4883 | 46604 |
| P/C ratio | 1.4 | 3.8 | 26.4 | 263.1 |

### (2-2) Storage stability test

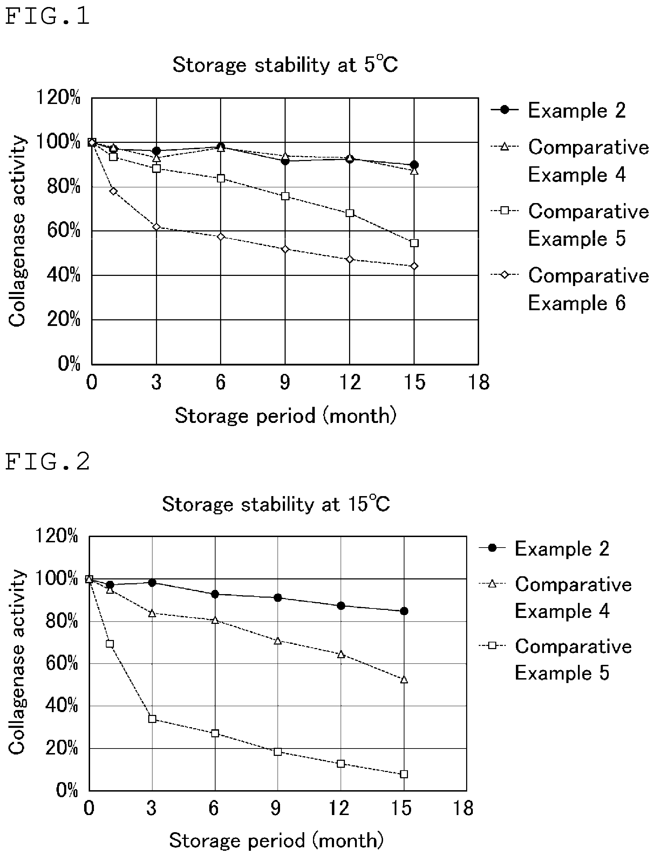

The enzyme compositions were stored at 5°C, 15°C, or 20°C for 15 months. The collagenase activity of the enzyme compositions were measured before and after storage as described in the section (1-2), and the relative activity was calculated based on the activity immediately after production, taken as 100%. FIG. 1 to FIG. 3 show the results.

As shown in FIG. 1 to FIG. 3, the residual activity significantly decreased at 15°C and 25°C in Comparative Examples 4 to 6. In Comparative Examples 5 and 6, the residual activity also significantly decreased at 5°C at which the reactivity of protease is low. In Example 2, the residual activity was maintained at 65% or higher after storage at 25°C for 15 months.

### (3) Example 3, Comparative Examples 7 and 8

### (3-1) Production of enzyme composition

The collagenase was added to a 50% glycerol solution such that it had the activity shown in Table 3 to produce a liquid enzyme composition. In Comparative Examples 7 and 8, the protease was also added in addition to the collagenase.

**[Table 3]**

| | Example 3 | Comparative Example 7 | Comparative Example 8 |
|---|---|---|---|
| Collagenase activity C (U/g) | 186 | 177 | 126 |
| Protease activity P (U/g) | 253 | 4651 | 32226 |
| P/C ratio | 1.4 | 26.2 | 255.5 |

### (3-2) Meat tenderizing test

The components shown in Table 4 were mixed with water to produce a pickling liquid containing the enzyme composition of Example 3, Comparative Example 7, or Comparative Example 8.

**[Table 4]**

| | % by weight |
|---|---|
| Enzyme composition | 0.5 |
| Sodium chloride | 1.5 |
| Sodium bicarbonate | 1 |
| Calcium lactate | 0.5 |

A piece of commercial pork loin was pre-treated using a Jaccard tenderizer. A red meat portion alone was cut out and divided into several 30 g fragments. Thirty grams of the pickling liquid containing the enzyme composition of Example 3, Comparative Example 7, or Comparative Example 8, or the corresponding pickling liquid without the enzyme composition (Reference Example 2), was dispensed into a bag. Then, two meat fragments (total 60 g) were placed in each bag and subjected to tumbling at 10°C for two hours. The samples after the tumbling were stored at 10°C.

The meat fragments after three days of storage at 10°C were baked in an oven at 200°C for 10 minutes (five minutes per face) and cooled to room temperature. Then, the fracture stress (N) was measured with a rheometer (SUN RHEO METER CR-500DX available from Sun Scientific Co., Ltd.). In Reference Example 1, the meat not treated with the pickling liquid was subjected to the same process to measure the fracture stress (N).

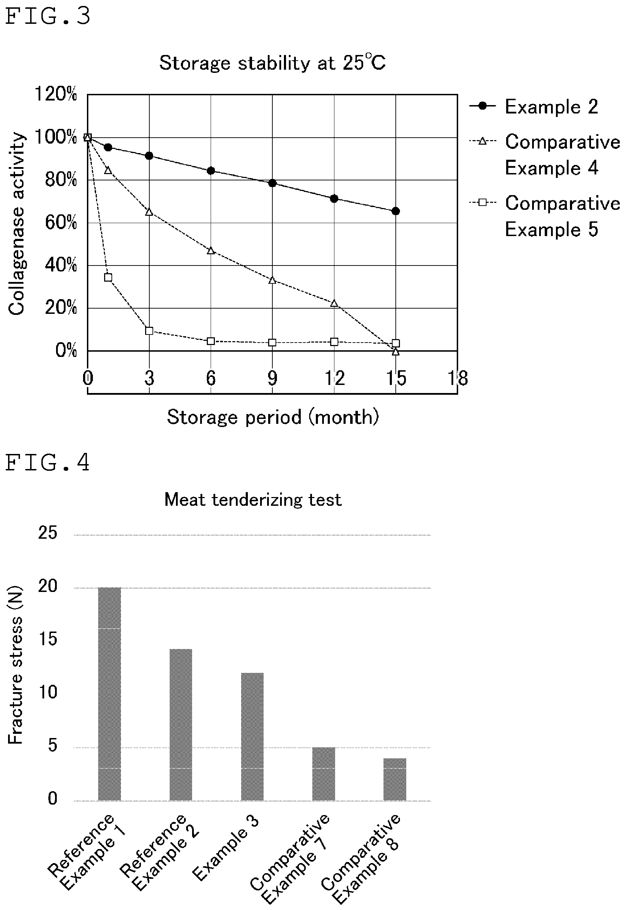

Generally, meat with a fracture stress of 10 to 20 N has an appropriate texture. As shown in FIG. 4, the meat treated with the pickling liquid containing the enzyme composition of Example 3 had an appropriate fracture stress. The meat treated with the pickling liquid containing the enzyme composition of Comparative Example 7 or 8 showed a decrease in fracture stress due to excessive tenderization of the meat.

Embodiment 1 of the present disclosure relates to an enzyme composition for producing a food, containing a collagenase and having substantially no contaminating activity.

Embodiment 2 of the present disclosure relates to the enzyme composition according to Embodiment 1 of the present disclosure,
wherein the contaminating activity is protease activity.

Embodiment 3 of the present disclosure relates to the enzyme composition according to Embodiment 1 or 2 of the present disclosure,
wherein the enzyme composition has a P/C ratio of 2.0 or lower, where P represents protease activity (U/g) and C represents collagenase activity (U/g).

Embodiment 4 of the present disclosure relates to the enzyme composition according to any one of Embodiments 1 to 3 of the present disclosure,
wherein the collagenase is derived from a bacterium.

Embodiment 5 of the present disclosure relates to the enzyme composition according to any one of Embodiments 1 to 4 of the present disclosure,
wherein a residual collagenase activity of the enzyme composition is 50% or higher after storage at 40°C for one month.

Embodiment 6 of the present disclosure relates to the enzyme composition according to any one of Embodiments 1 to 5 of the present disclosure,
wherein the food is a collagen peptide or a processed meat food.

Embodiment 7 of the present disclosure relates to a food production method, including processing a food material using the enzyme composition according to any one of Embodiments 1 to 6 of the present disclosure.

Embodiment 8 of the present disclosure relates a food, containing the enzyme composition according to any one of Embodiments 1 to 6 of the present disclosure.

## Claims

1. An enzyme composition for producing a food, comprising a collagenase and having substantially no contaminating activity.

2. The enzyme composition according to claim 1,
wherein the contaminating activity is protease activity.

3. The enzyme composition according to claim 1 or 2,
wherein the enzyme composition has a P/C ratio of 2.0 or lower, where P represents protease activity (U/g) and C represents collagenase activity (U/g).

4. The enzyme composition according to claim 1 or 2,
wherein the collagenase is derived from a bacterium.

5. The enzyme composition according to claim 1 or 2,
wherein a residual collagenase activity of the enzyme composition is 50% or higher after storage at 40°C for one month.

6. The enzyme composition according to claim 1 or 2,
wherein the food is a collagen peptide or a processed meat food.

7. A food production method, comprising processing a food material using the enzyme composition according to claim 1 or 2.

8. A food, comprising the enzyme composition according to claim 1 or 2.
